# EUROPEAN PATENT SPECIFICATION

(11) **EP 3 184 115 B1**
(45) Date of publication and mention of the grant of the patent: **05.12.2018**
(21) Application number: 16205086.8
(22) Date of filing: 19.12.2016
(51) Int. Cl.: A61K 36/48, A61P 1/04, A61K 9/00

(54) **ORAL COMPOSITIONS FOR THE TREATMENT OF GASTROESOPHAGEAL REFLUX DISEASE**
ORALE ZUSAMMENSETZUNGEN ZUR BEHANDLUNG VON GASTROÖSOPHAGEALEM REFLUX
COMPOSITION ORALE POUR LE TRAITEMENT DU REFLUX GASTRO- SOPHAGIEN

(30) Priority: 22.12.2015 IT UB20159732
(43) Date of publication of application: 28.06.2017
(73) Proprietor: S.I.I.T. S.r.l.-Servizio Internazionale Imballaggi Termosaldanti, 20090 Trezzano sul Naviglio MI (IT)
(72) Inventor: MADARO, Elena, 20090 TREZZANO SUL NAVIGLIO (MI) (IT); DOMINONI, Mirko, 20090 TREZZANO SUL NAVIGLIO (MI) (IT); MARCELLONI, Luciano, 20090 TREZZANO SUL NAVIGLIO (MI) (IT); COSTA, Andrea, 20090 TREZZANO SUL NAVIGLIO (MI) (IT)
(74) Representative: Bianchetti Bracco Minoja S.r.l.

(56) References cited:
- DE-A1- 10 027 050
- GB-A- 1 524 740
- S GUPTA ET AL: "Tamarind kernel gum: An upcoming natural polysaccharide", SYSTEMATIC REVIEWS IN PHARMACY, vol. 1, no. 1, 2010, pages 50-54, XP055282827, IND ISSN: 0975-8453, DOI: 10.4103/0975-8453.59512
- ALESSANDRA SEMENZATO ET AL: "Green Polymers in Personal Care Products: Rheological Properties of Tamarind Seed Polysaccharide", COSMETICS, vol. 2, no. 1, 23 December 2014 (2014-12-23), pages 1-10, XP055282832, DOI: 10.3390/cosmetics2010001
- SURESH KUMAR ET AL: "Antiulcer effect of the methanolic extract of Tamarindus indica seeds in different experimental models", JOURNAL OF PHARMACY AND BIOALLIED SCIENCES, vol. 3, no. 2, 1 January 2011 (2011-01-01) , pages 1-9, XP055282839, IN ISSN: 0975-7406, DOI: 10.4103/0975-7406.80778
- PAL DILIPKUMAR ET AL: "Novel tamarind seed polysaccharide-alginate mucoadhesive microspheres for oral gliclazide delivery: in vitro-in vivo evaluation.", DRUG DELIVERY APR 2012, vol. 19, no. 3, April 2012 (2012-04), pages 123-131, XP009190678, ISSN: 1521-0464
- PRANJAL SAIKIA ET AL: "Mucoadhesive nanoparticles from tamarind seed polysaccharides for sustained delivery of anticancer drug irinotecan", ASIAN JOURNAL OF PHARMACEUTICS, vol. 7, no. 4, 1 January 2013 (2013-01-01) , page 163, XP055282850, IN ISSN: 0973-8398, DOI: 10.4103/0973-8398.128884
- AMIT KUMAR NAYAK ET AL: "Ionotropically-gelled mucoadhesive beads for oral metformin HCl delivery: Formulation, optimization and antidiabetic evaluation", JOURNAL OF SCIENTIFIC & INDUSTRIAL RESEARCH, vol. 72, 1 January 2013 (2013-01-01), pages 15-22, XP055282867,

## Description

The present invention relates to an oral composition useful for the treatment of gastroesophageal reflux disease. The oral composition according to the invention comprises tamarind extract, alginic acid or a salt thereof, carbomer and a carbonate and/or hydrogen carbonate salt. Said composition is characterised by rapid action against the symptoms of reflux, and is particularly effective because it does not produce insoluble lumps in the acid environment of the stomach.

### Background to the invention

The production of gastric juices, which contain hydrochloric acid, digestive enzymes (pepsin) and mucus, is a physiological process necessary for the breakdown of foods, to make them available for subsequent absorption in the intestine. Healthy gastric mucosa is protected against acid, enzyme or mechanical attacks by an adherent layer of mucus, mainly consisting of mucin, water, cells, hydrogen carbonate and other salts. During digestion, the same stimuli that induce acid secretion simultaneously stimulate the production of a film of mucus. However, some particular conditions adversely influence the protective layer of mucus; for example, gastric secretion of a larger than normal amount of hydrochloric acid (hyperacidity), with or without dyspepsia, the use of medicaments or irritant foods (spices, coffee, alcohol, etc.), physical injury or mechanical damage, anxiety and stress, can reduce the protective capacity of the barrier and cause the gastric mucosa to come into direct contact with irritants. This leads to irritation of the mucosa, with symptoms such as pain, a burning sensation, post-prandial nausea and swelling, and can induce chronic gastritis and, in the last analysis, also ulcers of the gastric or duodenal wall.

Gastroesophageal reflux (GER) arises when the oesophageal sphincter opens spontaneously or fails to close for varying periods of time, causing the acidic gastric juices to rise, together with food, from the stomach to the oesophagus, and sometimes even to the mouth.

Gastroesophageal reflux syndrome (GERD) arises when the main episodes of reflux take place at least two or three times a week. This syndrome is due to incomplete closure of the cardiac sphincter at the top of the stomach, and symptoms range from a burning sensation to oesophagitis, nerve reflexes on the vagus nerve, thickening of the oesophageal area (hiatus hernia), and mutations of the epithelium (Barrett's oesophagus).

The disease can give rise to serious complications, especially a chronic inflammatory state (reflux oesophagitis) which, with time, can cause bleeding and alterations of the mucosa that narrow the oesophageal lumen and can make swallowing difficult.

GERD can also promote oesophageal cancer, which is preceded by alterations of the mucosa known as Barrett's oesophagus. Other studies demonstrate that GERD can also worsen or contribute to respiratory disorders (such as asthma, chronic cough, and pulmonary fibrosis). When a reflux of acids from the stomach comes into contact with the lining of the oesophagus, it not only causes direct damage to the mucosa, but can also cause a burning sensation in the chest and throat known as "heartburn".

In children, heartburn is rare, and the most frequent symptoms are a dry cough, asthma symptoms, or swallowing problems. The reason why some individuals develop GERD is not yet fully understood; in many cases it is associated with hiatus hernia, an otherwise asymptomatic anatomical abnormality wherein the upper part of the stomach and the oesophageal sphincter protrude above the diaphragm muscles.

Other factors that contribute to gastroesophageal reflux disease are more closely linked with lifestyle, such as obesity and frequent consumption of fried, spicy or acid foods or alcohol. Recent findings demonstrate that the pH of the reflux is often more acid than that of the concomitant intragastric pH. This apparent paradox has led to the discovery of the "acid pocket", a zone of unbuffered gastric acid that accumulates in the proximal stomach after meals and acts as a reservoir for acid reflux in predisposed individuals (Yamada, 2008, Kahrilas, 2013; Rohof, 2013). Medical treatment of gastric hyperacidity, oesophageal reflux and GERD can include administration of one or more of the following categories of OTC products or prescription drugs:
- antacids containing different combinations of magnesium, calcium or aluminium with hydroxide or hydrogen carbonate, to partly neutralise gastric acidity. The potential intestinal side effects of antacids are diarrhoea and constipation;
- prokinetics, which comprise all the substances that accelerate stomach voiding. This group comprises medicaments like bethanechol and metoclopramide, together with natural substances like ginger;
- histamine H2-receptor beta-blockers such as cimetidine, famotidine and ranitidine are medicaments that reduce acid production in the stomach;
- proton pump inhibitors such as omeprazole, esomeprazole and pantoprazole inhibit the production of gastric acid with a different action mechanism from histamine receptor blockers;
- prostaglandin analogues such as misoprostol, which inhibit acid production by bonding to the receptors on the EP3 parietal cells, and by stimulating mucin and hydrogen carbonate production;
- substances that stimulate production of the hydrogen carbonate ion in the stomach, such as carbenoxolone;
- transient lower oesophageal sphincter relaxation inhibitors (TLESR);
- foaming and cytoprotective agents such as Gaviscon, containing substances that form a foam which prevents episodes of reflux by coating the stomach and the lower oesophagus. They are usually combined with antacids, but can also be used in combination with prokinetics or proton pump inhibitors. Unlike the products belonging to other groups, these medical devices are practically devoid of side effects. Said medical devices are based on alginic acid and the salts thereof, namely sodium alginates.

Alginic acid is extensively present in the cell walls of brown algae. It is a linear copolymer with homopolymeric blocks of β-D-mannuronate (M) bonded with covalent bonds 1-4 to residues of its epimer α-L-guluronate (G) in different block sequences. The monomers can be homopolymeric blocks of consecutive G residues (G blocks) or blocks of consecutive M residues (M blocks), or blocks consisting of alternating M and G residues (MG blocks). In contact with water it forms a gel whose viscosity depends on concentration, pH and the presence of electrolytes.

Alginates have long been used as thickeners and stabilisers in the food and pharmaceutical industries.

The alginates described in GB1524740 form the basis of the product Gaviscon. In a strongly acid environment like the stomach, thick, neutral gels form which are able to float on the gastric juices and form a thick, dense layer called a "raft", which acts as a plug for gastroesophageal reflux and inhibits acid regurgitation from the stomach to the oesophagus.

The formation and characterisation of the raft are described in the article "Alginate rafts and their characterisation" (Hampson FC, Farndale A, Strugala V, Sykes J, Jolliffe IG, Dettmar PW).

Said study analyses the *in vitro* effectiveness of a range of liquid products in forming rafts that were cohesive, voluminous, resistant to reflux and durable under conditions of movement (resilient). The products tested had acid neutralisation capacities (ANCs). It was found that products with a high ANC and no calcium ion source formed rafts of low strength, weight and volume, which appeared more as floating precipitates than coherent gels. Products with a high ANC and a calcium ion source formed medium strength rafts. Products with a low ANC formed strong, coherent rafts with medium and high strength, and those with a low ANC and a calcium ion source formed the strongest rafts. Products with strong rafts were found to be more resistant to reflux in an *in vitro* model.

In parallel with the ANCs, the measurements taken relate to Strength and Resistance to Reflux by extrusion through an orifice of said rafts.

Table 1 shows the compositions, in terms of active ingredients, of some common commercial preparations based on alginates.

**Table 1**

| Product | Dose: ml or tablet | Alginic acid or Na alginate (mg/dose) | NaHCO₃ (mg/dose) | Other antacid ingredients | |
|---|---|---|---|---|---|
| | | | | Ingredient | (mg/dose) |
| Gaviscon Liquid (UK) | 10-20 mL | 500-1000 | 267-534 | CaCO₃ | 160-320 |
| Gaviscon Double Action (UK) | 10 mL | 500 | 213 | CaCO₃ | 325 |
| Gaviscon Advance (It) | 10 mL | 1000 | 200 (KHCO₃) | CaCO₃ | 200 |
| Gaviscon Advance (UK) | | | | | |
| Gaviscon Compresse (It) | 1-2 tablets | 500-1000 | 170-340 | AlOH₃ gel + Mg silicate | 100-200 |
| Gaviscon Tablets (UK) | | | | | 25-50 |
| Gaviscon Antacid (USA) | 2 tablets | n.d. | n.d. | AlOH₃ gel + Mg silicate | 160 |
| | | | | | 40 |
| Gaviscon Extra Strength (USA) | 2 tablets | n.d. | n.d. | AlOH₃ / MgCO₃ gel + MgCO₃ | 250 |
| | | | | | 160 |
| Gaviscon Liquid (USA) | 15 mL | n.d. | n.d. | AlOH₃ / MgCO₃ gel + MgCO₃ | 160 |
| | | | | | 392 |
| Gaviscon Extra Strength Liquid (USA) | 10 mL | n.d. | n.d. | AlOH₃ / MgCO₃ gel + MgCO₃ | 819 |
| | | | | | 409 |
| Gaviscon Tablets (Fr) | 1 tablets | 520 | 88.5 | AlOH₃ gel + | 167 |
| | | | | Mg silicate | 30 |
| Gaviscon Suspension (Fr) | 10 mL | 500 | 267 | CaCO3 + NaOH | |
| Gaviscon Infant (UK) | 1 sachet | 225 Na alg + 67.5 Mg alg. | - | - | - |
| Gastrocote (UK) | 2 tablets | 400 | 140 | AlOH3 gel + Mg silicate | 160 |
| | | | | | 80 |
| Algicon Liquid (UK) | 10 mL | 500 | 100 | AlOH3 / MgCO3 gel + MgCO3 | 280 |
| | | | | | 350 |
| Rennie Alginate (NL) | 10 mL | 300 | - | CaCO3 | 1200 |
| | | | | MgCO3 | 140 |

### Description of the invention

The invention is defined as set forth in the claims. All other embodiments are described for illustrative purposes only, and any statement that they would also be part of the invention or other, similar statements, have to be seen in the context of the application as filed and do not alter the scope of the claims.

In the anatomical area affected by GERD there is extreme variability of the dynamic conditions, and considerable irregularities in the constitution, disintegration and physiological resorption of the barrier layer called the "raft". The raft may be insufficiently adhesive and elastic to adhere constantly to the tissue of the oesophagus and cardia, in all their movements and variations of shape.

In some cases problems have been encountered in paediatric use; in particular the formation of an indigestible mass that persists *in situ* and is difficult to remove, called a "gaviscoma", has been reported.

Nevertheless, there is increasing demand for rapid relief of heartburn and reflux symptoms before the raft formed by the alginates acts on the oesophageal tissues; it would therefore be very useful to have an ingredient that coats the oesophagus and stomach with barrier properties characterised by rapid bioadhesion to the tissue.

The composition according to the present invention solves the problems and achieves the aims set out above, as it is characterised by rapid action at gastroesophageal level, providing immediate relief to the inflamed tissues and preventing the formation of the insoluble deposit that causes obstruction (gaviscoma).

The subject of the present invention is therefore an oral composition for use in the treatment of gastroesophageal reflux disease, comprising the following ingredients:
- tamarind extract
- alginic acid or a salt thereof
- a carbomer
- carbonate and/or hydrogen carbonate of a physiologically compatible alkali or alkaline-earth metal,
   wherein said tamarind extract is a dried aqueous or water-alcohol extract from the plant seeds having a titrated polysaccharide xyloglucan content of at least 30% by weight.

The tamarind plant (scientific name *Tamarindus indica*) is an evergreen belonging to the Leguminosae family, which can reach a height of 30 metres; its fruits are pods containing 4 to 12 seeds. Tamarind consists of 31% water, 57% sugars, 5% dietary fibres, ash, protein and fats; the minerals present in tamarind are potassium, present in large quantities, phosphorus, magnesium, sodium, calcium and selenium.

Tamarind contains vitamins A, B1, B2, B3, B5, B6, C, K, and J. It also contains a very small percentage of vitamin E.

The amino acids present include lysine, tryptophan and methionine. The acidic flavour of tamarind is due to its tartaric acid content of about 12%.

The properties of tamarind are partly due to dietary polysaccharides, hemicelluloses such as xyloglucan, pectins and mucilages, which immediately protect the surface of the irritated oesophagus on first pass. The xyloglucans extracted from tamarind seeds have been described as a viscosity booster with mucomimetic, mucoadhesive and bioadhesive activities (Saettone, 1997; Gupta, 2010, Semenzato, 2015).

The polysaccharide-rich tamarind extract is prepared by extraction of the seeds of the plant with water or water-alcohol solvent, preferably with water or water and ethanol in variable proportions. The resulting extract is then dried. Preferably the extract according to the invention has a titrated polysaccharide xyloglucan content of at least 75% by weight.

In a particularly preferred embodiment of the invention, the extract according to the invention takes the form of a yellow-white powder, and has the following characteristics:
- HPLC assay value of *Tamarindus indica* in xyloglucan, based on the dried substance >= 75 %
- Dynamic viscosity 500-2000 cPs (c = 2, water, 100 rpm)
- Weight loss on drying <= 15.0% (°C= +85, t= 16 hours)
- Total ash <= 3.0 % according to Eur. Pharm.

The composition according to the invention preferably contains 25 to 400 mg of said tamarind extract per dosage unit.

Alginic acid or the salt thereof used in the composition according to the invention generally has a viscosity below 2000 mPas at the concentration of 10% in water at 20°C-Brookfield Lv, 20 rpm. The composition preferably contains 25 to 900 mg of alginic acid or a salt thereof, more preferably 50 to 500 mg.

Carboxyvinylpolymer (or carboxypolymethylene), commercially known as carbomer (Handbook of Excipients, 5th Ed., p. 111) or Carbopol®, is a synthetic high-MW polymer obtained by polymerisation of acrylic acid, in the presence of 0.75-2% w/w of allyl saccharose to obtain a crosslinked structure.

The carboxyl group (-COOH) content ranges between 56.0 and 68.0%. The aqueous dispersion of the powder requires neutralisation with NaOH or organic bases to obtain gels with different viscosities. The viscosity is influenced by the pH. The pH in use generally ranges between 5 and 9. It gives rise to viscous solutions or gels, and the viscosity depends on the pH and the neutralising base.

A carbomer can be grade 934P, 971P or 974P, and is preferably Carbopol 974P, which presents a viscosity ranging between 25000 and 40000 cP at the concentration of 0.5% in water at pH 7.3-7.8 at 25°C-Brookfield RTV, spindle no. 6.20 rpm.

The composition according to the invention preferably contains 10 to 100 mg of carbomer per dosage unit.

The composition according to the invention also contains carbonate and/or hydrogen carbonate of a physiologically compatible alkali or alkaline-earth metal, preferably selected from calcium carbonate and potassium hydrogen carbonate. The composition preferably contains 10 to 500 mg of said carbonate and/or hydrogen carbonate of physiologically compatible alkali or alkaline-earth metal.

As further active ingredients, the composition can contain mallow extract, chamomile extract or ginger extract, in the form of essential oils, as dried extracts or fluids, or as plant material.

The composition according to the invention also contains excipients such as diluents, flavourings and sweeteners.

Said composition can be in solid or liquid form, preferably in the form of a tablet or an aqueous suspension.

The solid compositions generate a liquid suspension with the saliva after chewing, and preferably take the form of a water-soluble or orosoluble granulate or a chewable tablet. The liquid compositions preferably take the form of a multidose or single-dose vial or sachet.

Strong adhesion to the mucosa has been observed in animal models, comparable to that exerted by guar gum and greater than that of other neutral water-soluble mucoadhesive polymers such as arabinogalactans, hyaluronic acid, hydroxypropylcellulose, hydroxyethylcellulose and polyvinyl alcohol (Nakamura, 1996; Di Colo, 2009; Uccello-Barretta, 2011 and 2013).

The combination of ingredients therefore provides immediate protection for the oesophageal tissue due to an effective barrier effect.

An increase in the fluidity, plasticity and dissolution rate of the raft formed by alginates and basic carbonate salts was surprisingly observed during the product characterisation tests. Thus as well as providing a faster protective effect on the oesophagus and stomach due to the presence of tamarind polysaccharides, the composition according to the invention gives rise to better adhesion of the alginate raft under the different dynamic conditions of the oesophagus and cardia, and faster resorption of the alginate formation, thus preventing the deposit of undesirable masses at the entrance to the stomach.

The composition according to the invention therefore presents the following properties: (1) protective action on the mucosa of the stomach and oesophagus; (2) prevention of acidic foam formation in the stomach; (3) effective activity against gastritis and ulcers.

### Example 1 - chewable tablet

### Example 1A:

| INGREDIENT | (mg) Per dosage unit | g/100 g |
|---|---|---|
| *First group* | | |
| Calcium carbonate gran. Pharmagnesia CC Type 140 | 25.0 | 2.27 |
| Potassium hydrogen carbonate (fine powder) | 100.0 | 9.09 |
| *Tamarindus indica* xyloglucan | 50.0 | 4.55 |
| Sodium alginate | 200.0 | 18.19 |
| Microcrystalline cellulose 102 | 240.0 | 21.82 |
| Mannitol 200 SD | 126.4 | 11.49 |
| Mannitol CD 400 | 145.0 | 13.18 |
| Magnesium stearate | 10.0 | 0.91 |
| Maltodextrins IT19 | 171.2 | 15.56 |

| *Second group* | | |
|---|---|---|
| Mint flavouring | 15.0 | 1.36 |
| Sucralose | 3.0 | 0.27 |
| Levilite | 10.0 | 0.91 |
| Maltodextrins IT19 | 4.4 | 0.40 |

| | | |
|---|---|---|
| Characteristics of tablet: Round, flat, diameter 16 mm HARDNESS > 6-9 kN FRIABILITY < 3 % | | |

### Example 1B:

| INGREDIENT | (mg) Per dosage unit | g/100g |
|---|---|---|
| *First group* | | |
| Calcium carbonate | 25.000 | 2.62 |
| *Tamarindus indica* xyloglucan | 100.000 | 10.47 |
| Sodium alginate | 100.000 | 10.48 |
| Microcrystalline cellulose 102 | 150.000 | 15.71 |
| Mannitol 200 SD | 240.000 | 25.13 |
| Maltodextrins | 25.000 | 2.62 |

| *Second group* | | |
|---|---|---|
| Mint flavouring | 15.0 | 1.36 |
| Potassium hydrogen carbonate (fine powder) | 100.0 | 9.09 |
| Magnesium stearate | 10.000 | 1.05 |
| Sucralose | 2.360 | 0.25 |
| Maltodextrins | 171.200 | 17.93 |
| Milk flavouring | 10.000 | 1.05 |
| Aniseed flavouring | 10.000 | 1.05 |
| Levilite | 10.000 | 1.05 |

| | | |
|---|---|---|
| Characteristics of tablet: Diameter 14 mm HARDNESS > 5-7 kN FRIABILITY < 3% | | |

### Example 2 - Preparation process

a) In the case of flowable powders as in composition 1A (flow time in EP apparatus for measurement of dynamic flowability < 20 sec), use process of mixing and direct compression. Mix the ingredients listed in the first group of the following composition in a suitable powder mixer for 15 minutes at 3-10 rpm. Then add those of the second group and mix for a further 3 minutes. Compress the mixture with a suitably equipped tablet press;
b) In the case of non-flowable powders as in composition 1B, use a granulation process in a suitable mixer with liquid (water) poured on the excipients of the first group, and dry in a suitable dryer until the residual moisture is below 6%. Then add those of the second group and mix for a further 3 minutes. Press the mixture with a suitably equipped tablet press.

### Example 3 - ready-for-use liquid formulation

| INGREDIENT | (mg) Per dosage unit | g/100g |
|---|---|---|
| *First group* | | |
| Demineralised water | 3592.500 | 23.950% |
| Carbopol 974P | 45.000 | 0.300% |

| *Second group* | | |
|---|---|---|
| Demineralised water | 10500.000 | 70.000% |
| Heavy calcium carbonate | 50.000 | 0.333% |
| Sodium alginate | 400.000 | 2.667% |
| Nat. ginger | 2.400 | 0.016% |
| Essential oil of ginger | 2.100 | 0.014% |
| Condensed milk flavouring | 30.000 | 0.200% |
| Aniseed flavouring | 30.000 | 0.200% |
| Potassium hydrogen carbonate | 200.000 | 1.333% |
| Dried extract of tamarind | 100.000 | 0.667% |
| Nipagin sodium | 30.000 | 0.200% |
| Sucralose | 3.000 | 0.020% |
| Ethyl alcohol | 15.000 | 0.100% |
| | | |
| NaOH | q.s. | q.s. |
| *Specifications of suspension* | | |
| *Stick pack containing 15 ml (equivalent to about 15000 mg) of suspension* | | |
| *Appearance: a creamy liquid, colour cream to dark cream* | | |
| *pH: 8.20* +/- *0.2* | | |
| *Density: 1.024* +/- *0.05 g*/*mL* | | |

The product in this example, tested in a beaker containing 150 ml of 0.1N hydrochloric acid, has the following performance characteristics:
**Average Time** taken for the precipitate of product **to rise to the surface** of the hydrochloric acid (seconds)

| **Avg Time** (sec) | **SD** |
|---|---|
| 56.33 | ±3.72 |

**Average Thickness of the raft 30 seconds after** the addition of the product

| **Avg Thickness** (mm) | **SD** |
|---|---|
| 15.67 | ±2.07 |

**Average Thickness of the raft 2 minutes after** the addition of the product

| **Avg Thickness** (mm) | **SD** |
|---|---|
| 15.17 | ±1.47 |

The produced raft has been isolated and characterized for its visco-elasticity properties using the Visco rotational viscosimeter apparatus Rhostress 600, Haake, Enco, I.

The dynamic viscoelasticity (oscillation tests) apply a constant shear stress in the linear viscoelastic region, with increasing frequency in the range from 0.1 to 10 Hz.

The viscoelastic parameters considered were the storage modulus G' (storage modulus), index of elastic properties, and tgα.

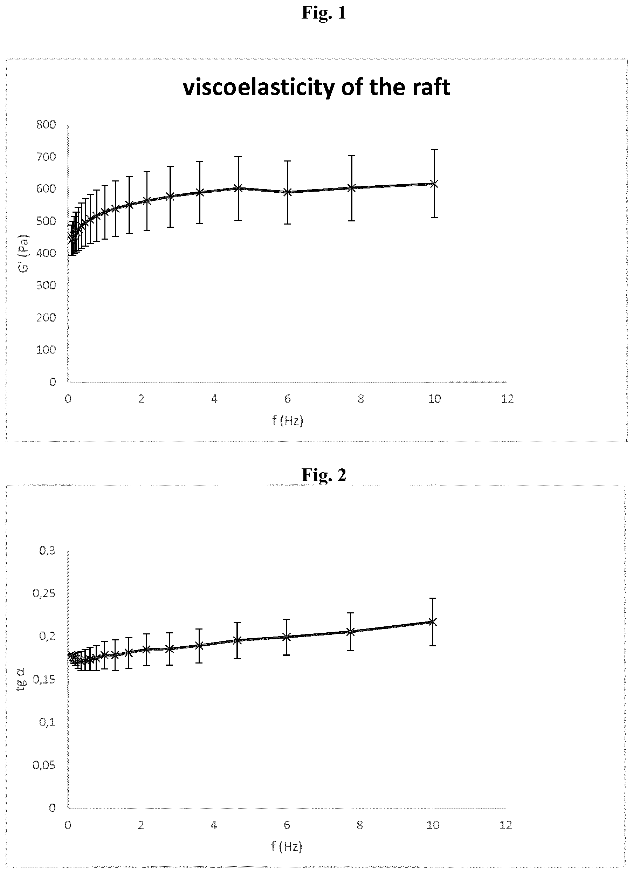

The results are reported in Figures 1 and 2, wherein:
Figure 1: Measurement of G' of the isolated raft vs the rate of the viscosimeter detection
Figure 2: Measurement of tgα of the isolated raft vs the rate of the viscosimeter detection

### Example 4 - Preparation process

In a first container disperse the carbomer in about 23% of the total water and maintain under slow stirring (stage 1). In a second container disperse the ingredients of the second group in water and maintain under slow stirring for an hour (stage 2).

Add stage 2 to stage 1 and adjust to a pH between 8 and 8.4 with NaOH molar solution 1 (or 30% sol.) as necessary.

Package the resulting suspension on a packaging line equipped to generate suitable containers (vials, stick packs) containing the product.

### Example 5 - Bioadhesion tests

The main objective of this research, conducted at the Catania University Department of Pharmaceutical Science, was to evaluate, in the first stage of the trial, the mucoadhesion *in vitro* of the liquid product (called Ginger Tamarind Anti-reflux PJ347-6) towards the epithelial cells of the gastrointestinal tract. The second objective, developed in the second stage of the trial, was to determine the resistance over time of the mucoadhesive layer (formed by interaction of the product with the mucosal cells) to the action of an acidic solution of HCl (pH 3.5), simulating the gastric juices.

The mucoadhesive capacity of the product Ginger Tamarind Anti-reflux (PJ347-6) was evaluated with an *in vitro* test using Caco-2 cell lines.

The intestinal cells of the Caco-2 line were selected as epithelial cell model because they present morphological and biochemical characteristics typical of the absorptive enterocyte present in the gastrointestinal tract. The bond of the substance was evaluated according to the lectin-glycoprotein percentage inhibition. Lectin is artificially bonded to biotin, which is able to bond with the streptavidin peroxidase added to the cell suspension. After repeated washes, O-phenyldiamine (O-Pd) is added to the cell suspension in the presence of hydrogen peroxide. If streptavidin peroxidase is present in the reaction medium, the O-Pd is oxidised, and a yellow colour develops. The reaction is stopped with 1M HCl after about a minute. The intensity of the colour, measured with a spectrophotometer reading, is proportional to the quantity of the lectin bond with the glucoside groups on the cell membrane.

In the case of cells pre-treated with a product having mucoadhesive capacity (incubation at 30°C for 15 minutes before treatment with lectin), the sites for the lectin bond are occupied by the test substance, causing a reduction in the intensity of the final colour because less streptavidin peroxidase bonds to the biotinylated lectin.

The results are expressed on the basis of the bond percentage of the mucoadhesive substance. The control cells are deemed to be 100% of the coloured cells. The fewer cells are coloured (as a percentage of the control cells), the higher the quantity of mucoadhesive substance bonded to the cell membrane.

By way of reference, a good bioadhesive seldom exceeds 70% of the result.

Each experiment, relating to a given time of exposure to the flow of the acidic solution, was conducted in triplicate.

The results demonstrated good mucoadhesion of the product to Caco-2 gastrointestinal cells. The degree of mucoadhesion obtained for the solution of the Ginger Tamarind Anti-reflux (PJ347-6) product (diluted 1:2) was 69.6 ± 3.0%, significantly different from that obtained with the 1:5 dilution (56.6 ± 4.7 %, p < 0.05) (Table 2).

**Table 2**

| Percentage mucoadhesion of tamarind product (PJ347-6) (diluted 1:2 and 1:5) to Caco-2 gastrointestinal cells. | | |
|---|---|---|
| *Ginger Tamarind Anti- reflux (PJ347-6)* | **1:2 dilution** | **1:5 dilution** |
| A | 72.7 | 61.9 |
| B | 66.8 | 52.8 |
| C | 69.3 | 55.1 |
| Mean ± S.D. | 69.6 ± 3.0 | 56.6 ± 4.7 |

The second stage of the trial relates to determination of the strength of the mucoadhesive layer formed by interaction between the polysaccharide constituents of the products and the mucosal cells.

The results obtained in the dynamic tests indicated an interesting ability of these polysaccharides to remain bioadherent to the mucosa, ensuring good mucoadhesion for the first hour of contact, which still remained appreciable after the second hour.

After 0.5 h, 1 h and 2 h, the mucoadhesion percentages were 53.9 ± 4.0% (0.5 h), 46.0 ± 2.5 % (1 h); 37.0 ± 2.2 % (2 h) (Table 3)

**Table 3**

| Resistance of mucoadhesive layer obtained with the product Ginger Tamarind Anti-reflux (PJ347-6) diluted 1:5, at different times (0.5 h, 1 h and 2 h), to simulated gastrointestinal juices (HCl sol. pH 3.5). | | | | |
|---|---|---|---|---|
| *Ginger Tamarind Anti- reflux (PJ347-6)* | **0 hours** | **0.5 hours** | **1 hour** | **2 hours** |
| A | 61.9 | 58.3 | 47.1 | 37.8 |
| B | 52.8 | 50.7 | 43.2 | 34.5 |
| C | 55.1 | 52.6 | 47.8 | 38.6 |
| Mean ± S.D. | 56.6 ± 4.7 | 53.9 ± 4.0 | 46.0 ± 2.5 | 37.0 ± 2.2 |

### Example 6 - Rafting test with and without tamarind extract.

A simple *in vitro* method that detects raft formation is described in the article by C. Scarpignato and V. Savarino in Therapy perspectives Year XIV, no. 1, March 2011.
1. A narrow-neck Erlenmeyer flask calibrated to 100 ml is filled with an 0.1 N solution of hydrochloric acid;
2. 10 grams of "Anti-reflux" product are added from a stick pack;
3. The product is initially deposited on the base, forming a gel. Subsequently, the development of CO2 causes the material to float, and it simultaneously forms a gel and reaches the neck of the Erlenmeyer flask forming a viscous plug which, until holed, prevents the contents from exiting for a certain time.

The efficacy of said gel is established by inverting the Erlenmeyer flask and measuring the time taken for a hole to form in the gel and the liquid to exit.

The danger of residues such as the formation called a "gaviscoma" is evaluated on the basis of the time they remain after emptying of the lumpy gel remaining in the Erlenmeyer flask.

The following comparative data relate to the composition according to the invention (alginate, calcium carbonate, carbopol and tamarind extract), and were determined after its inversion in the Erlenmeyer flask with 0.1N hydrochloric acid. The comparison products evaluated were Gaviscon Advance (UK), which does not contain tamarind extract, and Gaviscon Liquid (UK), which contains neither carbopol nor tamarind extract.

| **Product** | **RAFT CREATION TIME (sec)** | | | **RAFT BREAKAGE TIME (sec)** | | | **PRESENCE OF RESIDUE IN ERLENMEYER FLASK AFTER EMPTYING** | | | **RAFT Homogeneity/Appearance** |
|---|---|---|---|---|---|---|---|---|---|---|
| **Sample no.** | **1** | **2** | **3** | **1** | **2** | **3** | **1** | **2** | **3** | |
| **GAVISCON ADVANCE**® | *90* | *85* | *120* | ***1*** | ***1*** | ***1*** | *YES* | *YES* | *YES* | **Nonhomogeneous with large lumps** |
| **GAVISCON LIQUID** | *120* | *130* | *95* | ***3*** | ***5*** | ***5*** | *NO* | *NO* | *NO* | |
| **Formula of example 3 without tamarind** | *35* | *40* | *37* | ***0.5*** | ***1*** | ***1*** | *NO* | *NO* | *NO* | **Filamentous, homogeneous** |
| **Formula of example 3 with tamarind** | *5* | *4* | *5* | ***3*** | ***3*** | ***5*** | *NO* | *NO* | *NO* | |

The results demonstrate greater plasticity in the presence of tamarind and an absence of lumpy residues after emptying of the Erlenmeyer flask.

The same test was repeated on the 2 best-performing samples of Gaviscon Liquid and the formula of example 3 with tamarind, but incubated for 30 minutes at a temperature of + 37°C, equivalent to body temperature.

| **Product** | **RAFT BREAKAGE TIME after 30 min at +37°C (sec)** | | | | **PRESENCE OF RESIDUE IN ERLENMEYER FLASK AFTER EMPTYING** | | | **RAFT Homogeneity/Appearance** |
|---|---|---|---|---|---|---|---|---|
| **Sample no.** | | **1** | **2** | **3** | **1** | **2** | **3** | |
| **GAVISCON LIQUID** | | *3* | *3* | *4* | *YES* | *YES* | *YES* | Nonhomogeneous with large lumps |
| **Formula of example 3 with tamarind** | | *10* | *8* | *7* | *NO* | *NO* | *NO* | Uniform and homogeneous |

At +37°C the product containing tamarind was always lump-free, thickened and more breakage-resistant than at room temperature.

## Claims

1. An oral composition for use in the treatment of gastroesophageal reflux disease comprising the following ingredients:
- tamarind extract
- alginic acid or a salt thereof
- a carbomer
- physiologically-compatible alkali or alkaline earth metal carbonate and/or hydrogen carbonate,
wherein said tamarind extract is a dried aqueous or water-alcohol extract from the plant seeds having a titrated polysaccharide xyloglucan content of at least 30% by weight.

2. A composition for use according to claim 1, wherein said tamarind extract has a titrated polysaccharide xyloglucan content of at least 75% by weight.

3. A composition for use according to claims 1-2, containing 25 mg to 400 mg per dosage unit of said tamarind extract.

4. A composition for use according to claims 1 and 3, containing 25 mg to 900 mg per dosage unit of alginic acid or a salt thereof.

5. A composition for use according to claim 4, containing 50 mg to 500mg per dosage unit of alginic acid or a salt thereof.

6. A composition for use according to claim 1, containing 10 mg to 100 mg per dosage unit of carbomer.

7. A composition for use according to claim 1, containing calcium carbonate and/or potassium hydrogen carbonate.

8. A composition for use according to claim 1, containing 10 mg to 500 mg per dosage unit of said physiologically-compatible alkali or alkaline earth metal carbonate and/or hydrogen carbonate.

9. A composition for use according to any one of the preceding claims comprising, as further active ingredients, mallow extract, chamomile extract or ginger extract.

10. A composition for use according to any one of the preceding claims, which is in solid or liquid form.

11. A composition for use according to claim 10, which is in the form of tablet or aqueous suspension.

## Patentansprüche

1. Orale Zusammensetzung zur Verwendung in der Behandlung von gastroösophagealer Reflux-Krankheit, umfassend die folgenden Ingredienzien:
- Tamarindenextrakt,
- Alginsäure oder ein Salz davon,
- ein Carbomer,
- physiologisch kompatibles Alkali- oder Erdalkalimetallcarbonat und/oder -hydrogencarbonat,
wobei der Tamarindenextrakt ein getrockneter wässriger oder Wasser-Alkohol-Extrakt aus den Pflanzensamen, die eine titrierten Polysaccharid-Xyloglucan-Gehalt von wenigstens 30 Gew.-% haben, ist.

2. Zusammensetzung zur Verwendung gemäß Anspruch 1, wobei der Tamarindenextrakt einen titrierten Polysaccharid-Xyloglucan-Gehalt von wenigstens 75 Gew.-% hat.

3. Zusammensetzung zur Verwendung gemäß Ansprüchen 1-2, die 25 mg bis 400 mg des Tamarindenextraktes pro Dosierungseinheit enthält.

4. Zusammensetzung zur Verwendung gemäß Ansprüchen 1 und 3, die 25 mg bis 900 mg Alginsäure oder ein Salz davon pro Dosierungseinheit enthält.

5. Zusammensetzung zur Verwendung gemäß Anspruch 4, die 50 mg bis 500 mg Alginsäure oder ein Salz davon pro Dosierungseinheit enthält.

6. Zusammensetzung zur Verwendung gemäß Anspruch 1, die 10 mg bis 100 mg Carbomer pro Dosierungseinheit enthält.

7. Zusammensetzung zur Verwendung gemäß Anspruch 1, die Calciumcarbonat und/oder Kaliumhydrogencarbonat enthält.

8. Zusammensetzung zur Verwendung gemäß Anspruch 1, die 10 mg bis 500 mg des physiologisch kompatiblen Alkali- oder Erdalkalimetallcarbonats und/oder -hydrogencarbonats pro Dosierungseinheit enthält.

9. Zusammensetzung zur Verwendung gemäß einem der vorangehenden Ansprüche, die als weitere aktive Ingredienzien Malvenextrakt, Kamillenextrakt oder Ingwerextrakt umfasst.

10. Zusammensetzung zur Verwendung gemäß einem der vorangehenden Ansprüche, die in fester oder flüssiger Form ist.

11. Zusammensetzung zur Verwendung gemäß Anspruch 10, die in der Form einer Tablette oder einer wässrigen Suspension ist.

## Revendications

1. Composition orale destinée à être utilisée dans le traitement de la maladie du reflux gastro-oesophagien comprenant les ingrédients suivants :
- extrait de tamarin
- acide alginique ou un de ses sels
- un carbomère
- carbonate métallique alcalin ou alcalino-terreux physiologiquement compatible et/ou hydrogénocarbonate,
où ledit extrait de tamarin est un extrait aqueux ou d'eau-alcool anhydre des graines de plante ayant une teneur en polysaccharide xyloglycane titrée d'au moins 30 % en poids.

2. Composition destinée à être utilisée selon la revendication 1, dans laquelle ledit extrait de tamarin a une teneur en polysaccaride xyloglucane titrée d'au moins 75 % en poids.

3. Composition destinée à être utilisée selon les revendications 1 à 2, contenant 25 mg à 400 mg par unité de dosage dudit extrait de tamarin.

4. Composition destinée à être utilisée selon les revendications 1 et 3, contenant 25 mg à 900 mg par unité de dosage d'acide alginique ou d'un de ses sels.

5. Composition destinée à être utilisé selon la revendication 4, contenant 50 mg à 500 mg par unité de dosage d'acide alginique ou d'un de ses sels.

6. Composition destinée à être utilisée selon la revendication 1, contenant 10 mg à 100 mg par unité de dosage de carbomère.

7. Composition destinée à être utilisée selon la revendication 1, contenant du carbonate de calcium et/ou de l'hydrogénocarbonate de potassium.

8. Composition destinée à être utilisée selon la revendication 1, contenant 10 mg à 500 mg par unité de dosage dudit carbonate métallique alcalin ou alcalino-terreux physiologiquement compatible et/ou hydrogénocarbonate.

9. Composition destinée à être utilisée selon l'une quelconque des revendications précédentes comprenant, comme autres principes actifs, de l'extrait de mauve, de l'extrait de camomille ou de l'extrait de gingembre.

10. Composition destinée à être utilisée selon l'une quelconque des revendications précédentes, qui est sous forme solide ou liquide.

11. Composition destinée à être utilisée selon la revendication 10, qui est sous forme de comprimé ou de suspension aqueuse.
